# EUROPEAN PATENT SPECIFICATION

(11) **EP 0 965 541 B1**
(45) Date of publication and mention of the grant of the patent: **05.01.2005**
(21) Application number: 99304787.7
(22) Date of filing: 18.06.1999
(51) Int. Cl.: B65F 1/14, A61L 11/00, A61L 9/01, A61L 2/16

(54) **Waste disposal system**
Abfallverarbeitungssystem
Système de traitement de déchets

(30) Priority: 18.06.1998 GB 9813207
(43) Date of publication of application: 22.12.1999
(73) Proprietor: Biotal Limited, Cardiff CF24 5PD (GB)
(72) Inventor: Caunt, Philip, Cardiff, CF4 5DL (GB); Williams, Deborah, Cardiff, CF4 5DL (GB)
(74) Representative: Perry, Robert Edward

(56) References cited:
- EP-A- 0 722 659
- WO-A-98/22152
- GB-A- 2 274 394
- GB-A- 2 314 025
- JP-A- 59 227 802
- US-A- 3 159 536
- US-A- 4 164 561
- PATENT ABSTRACTS OF JAPAN vol. 015, no. 128 (C-0818), 28 March 1991 (1991-03-28) & JP 03 012165 A (UBE IND LTD), 21 January 1991 (1991-01-21)
- DATABASE WPI Section Ch, Week 198926 Derwent Publications Ltd., London, GB; Class D22, AN 1989-190021 XP002141105 & JP 01 129854 A (NISSAN CHEM IND LTD), 23 May 1989 (1989-05-23)

## Description

### Field of the Invention

This invention relates to a waste disposal system comprising a bin that has been treated to prevent proliferation of microorganisms.

### Background to the Invention

Feminine hygiene waste, such as used sanitary towels and tampons, and soiled nappies, are often disposed of in specialist bins. There are a number of companies which in turn offer a service relating to these bins. In some cases, the bin is collected after a service interval and replaced by an empty bin. The used bin is then taken to a central depot for disposal of the waste, and the bins are cleaned before being used in further service visits. Other companies remove the waste on site in a liner, then clean the bin and replace the liner.

There is concern about the proliferation of microbes within the bin, and it is felt that this may present a hazard to the customers and operatives of the service companies, and may also lead to the development of unpleasant odours. To combat this, a biocidal system is often used in the bin. Traditionally, this has been a liquid disinfectant, e.g. containing quaternary ammonium compounds, but this leads to an increased weight of material requiring disposal, and there are also concerns regarding effectiveness ofa liquid system throughout the bin once the material has been absorbed into the sanitary waste at the base. Ways of overcoming this include the use of gas-generating systems which produce, for example, sulphur dioxide which can then penetrate and disinfect waste throughout the bin. There is some doubt about the control of release of the gas, as well as health and safety concerns about sulphur dioxide.

Up to 1 litre of a liquid disinfectant has traditionally been used in each feminine hygiene bin. The disinfectant contributes significantly to the waste disposal problem and its cost. Thus, a low volume of liquid or a small volume of a solid would be preferable. However, there are clearly issues relating to the ability of a low volume of sanitising material to penetrate through the large quantity of sanitary waste which may be present in a bin.

In the field of disinfection, there is a general move away from chemical agents, due to concerns regarding the effects of these chemical compounds, or their residues, on the environment. This has led to increased interest in the use of natural compounds as disinfectants in certain sectors. A number of essential oils and plant extracts are known to be microbiocidal, for example eucalyptus oils, tea tree oil, pine oils and garlic extract.

The anti-microbial nature of essential oils is well documented. Many have been used as traditional remedies for a number of years, and recently there has been an increase in interest in the use of essential oils in personal care products. Most of these are liquid or semi-solid products. The use ofessential oil vapours to disinfect various systems is also well understood (see, for example, GB-A-2314025).

US-A-4 164 561 discloses the use of essentiel oils and carriers for deodorising waste bins.

Patent Abstracts of Japan vol. 015, no. 128 (C-D818) discloses the use of tea tree oil for deodorising and sterilising waste.

### Summary of the Invention

According to one aspect of the present invention, a product for sanitising and deodorising a waste disposal bin, comprises tea tree oil absorbed onto a solid carrier of silicon dioxide. The product may be contained in any suitable dispensing device and a preferred device is a perforated plastics sachet.

According to another aspect, a waste disposal bin comprises therein a product for sanitising and deodorising the bin, wherein the product comprises a natural biocide absorbed onto a solid carrier of silicon dioxide.

### Description of the Invention

The solid carrier is of silicon dioxide. Amorphous silicon dioxide, by way of example, has the desirable properties of extremely small particle sizes (e.g. about 8 µm) and a high surface area to weight ratio (greater than 450 m²/g). The use of this material provides a means to effect the contact ofa small amount of active ingredient with a large volume of waste. A further advantage ofusing silicon dioxide is that products of this type have been reported as being lethal to a range of bacteria, perhaps by a desiccation mechanism, so that the carrier itself may also contribute to the anti-microbial nature of the system.

Any suitable natural biocide may be used. A natural oil such as tea tree oil is preferred. Experiments have shown that the volatile nature of natural materials such as essential oils means that bacteria can be killed by the action of the oil vapour.

A bin of the invention may include a multi-dosing unit, activated by the opening and closing of the bin lid. Such a unit may dose a measured quantity of the active ingredient onto each piece of waste as it is placed in the bin. Suitable means for activating such a unit, and delivering the ingredient, will be readily apparent to those skilled in the art. A preferred system comprises a lid that incorporates a double flap design. As the lid is lifted, a flap perpendicular to the lid is raised to the top of the bin, thus hiding the contents of the bin from the view of the user. As the lid closes, the flap falls back into a vertical position at the back of the bin. Movement of the lid, and particularly the lower flap, is used to activate a dispensing device. The preferred device is a perforated dispensing sachet, e.g. of a plastics material, which is located on the inside wall of the bin, behind the vertical flap; as the flap falls back, into the vertical position, after use, the sachet is squeezed, pushing a small dose of the active ingredient through the perforations. The active ingredient then falls onto the freshly deposited waste and carries out the disinfection and deodorisation.

A preferred embodiment of the sachet has approximate dimensions of 250 mm by 50 mm and, with the back seam, of approximately 15 mm length, laying flat along the back, which gives the sachet a spring-like ability to re-inflate itself, ready for subsequent use. The sachet comprises one or more sets of holes through which the product may be dispensed, and which allow ingress of air following the dispensing phase. The holes may be covered during supply, for instance by a removable tape which is removed once the sachet is installed. The sachet may be held in place horizontally in the bin either by means of a suitable adhesive, such as double-sided sticky tape, or by means of a hanging device which fits under the back seam.

The size of the sachet, the amount of silicon dioxide/tea tree oil mixture and the proportion oftea tree oil in the mixture can be altered depending on the number of doses required and the final use, such as feminine hygiene disposal or nappy bins.

The following Examples illustrate the invention.

### Example 1

Various natural anti-microbial agents such as garlic extract, eucalyptus oil, orange seed extract and tea tree oil were absorbed onto silicon dioxide at a level of 20% (w/w), and then tested for their ability to kill a test bacterium. A 5 ml aliquot of an overnight culture of *Salmonella spp* was allowed to soak into the upper surface of a number of sanitary towels. The test mixtures were applied to the impregnated towels by sprinkling 0.4 g of each onto the upper surface of individual towels. The towels were then placed in plastics boxes with loose-fitting lids and stored at room temperature for 7 days. The surviving bacteria were counted using standard microbiological techniques. The results showed that, of the mixtures tested, the tea tree oil and silicon dioxide mixture showed the greatest ability to kill bacteria absorbed into sanitary towels. Similar results were obtained for the ability of the tea tree oil and silicon dioxide mixture to kill other test microbes, such as *Pseudomonas, E. coli* and *Candida.* Tea tree oil is thus the preferred material for use in this invention.

### Example 2

Sanitary towels were inoculated with bacteria, dosed with the tea tree oil and silicon dioxide mixture described in Example 1, and then placed in the plastics boxes at room temperature, as in Example 1. At various time intervals, towels were removed from the boxes, and the surviving bacteria were counted. The results showed that the effect of the mixture is not immediate, and that more than 5 days' contact time with the biocidal mixture is required. However, between 5 and 8 days, the number of bacteria fell very sharply, to below the detection limit.

### Example 3

Experiments were conducted to test the effect of varying the total amount of mixture used and to study the effect of changing the percentage of oil present in the silicon dioxide. Experiments were carried out using the methods described above, and the towels were left at room temperature for 30 days. The results showed that it is possible to control bacterial numbers with halfthe original amount oftea tree oil, and that this can be done both by reducing the size of the dose whilst keeping the concentration the same, or by reducing the percentage of tea tree oil in a similar sized dose.

### Example 4

A bin was constructed, with a dispensing system as described above. Sachets were made of polyethylene, and the dosing technology was tested, with repeated use.

Each day, towels were numbered with the elapsed time for the experiment, inoculated with bacteria as before, and then placed in the bin as in normal use. As each towel was placed into the bin, the movement of the lid caused a small dose of the active ingredient mixture to be released onto the waste. At the end of the experiment (35 days), towels were removed, and a number, representing varying contact times with the anti-microbial system, were analysed for numbers ofsurviving bacteria. A control bin received no treatment, to check the long-term survival of the bacteria in this environment.

The results showed that the dosing system had worked effectively, in that no microbes were detectable on the towels in the treated bin throughout the duration of the trial, compared to counts consistently around 5 x 10⁵ colony-forming units/towel on towels in the control bin. It was interesting to note that the towel which had been in contact with the disinfecting system for only one day still had significant levels of microbes, confirming the results found earlier that between five and eight days' contact time is required for the antimicrobial effect.

In terms of microbiology, prior art technology resulted in a wide range of Gram-negative bacteria counts (using MacConkey agar). The material from the base of the bins had typically been soaked in the liquid disinfectant and, as a result, microbial counts from these samples were around 6 x 10² to 3 x 10⁴ colony-forming units/towel. However, higher up in the bins, total counts between 1 x 10⁸ and 9 x 10⁹ colony-forming units/towel were encountered. When using the present invention, there was no such variation ofmicrobe numbers, with counts ofbetween 6 x 10⁴ and 9 x 10⁶ colony-forming units/towel from all samples analysed. This showed that the new system was capable of consistently reducing the numbers of potentially hazardous Gram-negative bacteria throughout the use cycle of the bin.

### Example 5

A Sanitary towel was inoculated with bacteria as before, and then suspended in a jar above, and not touching, a Petri dish containing 1 ml of tea tree oil absorbed onto amorphous silicon dioxide. The jar was sealed and stored at room temperature for 20 days. At the end of the experiment, the number of surviving bacteria in the towel was measured. In a control towel which was not exposed to tea tree oil, 9 x 10⁷ colony-forming units/towel were recovered, whilst in the towel exposed to the tea tree oil preparation, no bacteria were detectable.

Experiments have also shown that the all the effects described are common for both so-called 'standard' and 'pharmaceutical' grades of tea tree oil.

## Claims

1. A waste disposal bin comprising therein a product for sanitising and deodorising the bin, wherein the product comprises a natural biocide absorbed onto a solid carrier **characterised in that** the solid carrier is of silicon dioxide.

2. A bin according to claim 1, wherein the solid carrier is amorphous silicon dioxide.

3. A bin according to claim 1 or claim 2, wherein the biocide is tea tree oil.

4. A bin according to any preceding claim, wherein the product is contained within a dispensing device.

5. A bin according to claim 4, wherein the dispensing device is a perforated plastics sachet.

6. A bin according to claim 4 or claim 5, wherein the dispensing device is a multi-dosing unit, activated by the opening and closing of a lid on the bin to thereby release the biocide.

7. A product for sanitising and deodorising a waste disposal bin, comprising tea tree oil absorbed onto a solid carrier **characterised in that** the solid carrier is of silicon dioxide.

8. A product according to claim 7, wherein the silicon dioxide is amorphous.

9. A perforated plastics sachet containing a natural biocide absorbed onto a solid carrier **characterised in that** the solid carrier is of silicon dioxide.

10. A sachet according to claim 9, wherein the silicon dioxide is amorphous.

11. A sachet according to claim 9 or claim 10, wherein the biocide is tea tree oil.

12. Use of a natural biocide to sanitise and deodorise a waste disposal bin, wherein the biocide is absorbed onto a solid carrier **characterised in that** the solid carrier is of silicon dioxide.

13. Use according to claim 12, wherein the silicon dioxide is amorphous.

14. Use according to claim 12 or claim 13, wherein the biocide is tea tree oil.

15. Use of a product or sachet according to any of claims 7 to 11, to prevent the proliferation of microorganisms within a waste disposal bin.

## Patentansprüche

1. Abfallentsorgungsbehälter, darin umfassend ein Produkt zum Hygienisch-machen und Deodorieren des Behälters, wobei das Produkt ein natürliches Biozid umfasst, das auf einem festen Träger absorbiert ist, **dadurch gekennzeichnet, dass** der feste Träger aus Siliciumdioxid ist.

2. Behälter nach Anspruch 1, bei dem der feste Träger amorphes Siliciumdioxid ist.

3. Behälter nach Anspruch 1 oder nach Anspruch 2, bei dem das Biozid Teebaumöl ist.

4. Behälter nach irgendeinem vorangehenden Anspruch, bei dem das Produkt innerhalb einer Abgabevorrichtung enthalten ist.

5. Behälter nach Anspruch 4, bei dem die Abgabevorrichtung ein perforierter Kunststoffbeutel ist.

6. Behälter nach Anspruch 4 oder Anspruch 5, bei dem die Abgabevorrichtung eine Multidosierungseinheit ist, die durch das Öffnen und Schließen eines Deckels auf dem Behälter aktiviert wird, wodurch das Biozid freigesetzt wird.

7. Produkt zum Hygienisch-machen und Deodorieren eines Abfallentsorgungsbehälters, umfassend Teebaumöl, das auf einem festen Träger absorbiert ist, **dadurch gekennzeichnet, dass** der feste Träger aus Siliciumdioxid ist.

8. Produkt nach Anspruch 7, bei dem das Siliciumdioxid amorph ist.

9. Perforierter Kunststoffbeutel, enthaltend ein natürliches Biozid, das auf einem festen Träger absorbiert ist, **dadurch gekennzeichnet, dass** der feste Träger aus Siliciumdioxid ist.

10. Beutel nach Anspruch 9, bei dem das Siliciumdioxid amorph ist.

11. Beutel nach Anspruch 9 oder Anspruch 10, bei dem das Biozid Teebaumöl ist.

12. Verwendung eines natürlichen Biozids, um einen Abfallentsorgungsbehälter hygienisch zu machen und zu deodorieren, wobei das Biozid auf einem festen Träger absorbiert ist, **dadurch gekennzeichnet, dass** der Träger aus Siliciumdioxid ist.

13. Verwendung nach Anspruch 12, bei der das Siliciumdioxid amorph ist.

14. Verwendung nach Anspruch 12 oder Anspruch 13, bei der das Biozid Teebaumöl ist.

15. Verwendung eines Produkts oder Beutels nach irgendeinem der Ansprüche 7 bis 11, um die Proliferation von Mikroorganismen innerhalb eines Abfallentsorgungsbehälters zu verhindern.

## Revendications

1. Bac de réception de déchets, contenant un produit pour désinfecter et désodoriser le bac, dans lequel le produit comprend un biocide naturel absorbé sur un support solide, **caractérisé en ce que** le support solide est de l'oxyde de silicium.

2. Bac selon la revendication 1, dans lequel le support solide est de l'oxyde de silicium amorphe.

3. Bac selon la revendication 1 ou la revendication 2, dans lequel le biocide est de l'huile d'arbre à thé.

4. Bac selon l'une quelconque des revendications précédentes, dans lequel le produit est contenu dans un dispositif de distribution.

5. Bac selon la revendication 4, dans lequel le dispositif de distribution est un sachet en plastique perforé.

6. Bac selon la revendication 4 ou la revendication 5, dans lequel le dispositif de distribution est une unité à plusieurs doses, activée par l'ouverture et la fermeture d'un couvercle sur le bac, pour libérer le biocide.

7. Produit pour désinfecter et désodoriser un bac de réception de déchets, comprenant de l'huile d'arbre à thé absorbée sur un support solide, **caractérisé en ce que** le support solide est de l'oxyde de silicium.

8. Produit selon la revendication 7, dans lequel l'oxyde de silicium est amorphe.

9. Sachet en plastique perforé contenant un biocide naturel absorbé sur un support solide, **caractérisé en ce que** le support solide est de l'oxyde de silicium.

10. Sachet selon la revendication 9, dans lequel l'oxyde de silicium est amorphe.

11. Sachet selon la revendication 9 ou la revendication 10, dans lequel le biocide est de l'huile d'arbre à thé.

12. Utilisation d'un biocide naturel pour désinfecter et désodoriser un bac de réception de déchets, dans laquelle le biocide est absorbé sur un support solide, **caractérisée en ce que** le support solide est de l'oxyde de silicium.

13. Utilisation selon la revendication 12, dans laquelle l'oxyde de silicium est amorphe.

14. Utilisation selon la revendication 12 ou la revendication 13, dans laquelle le biocide est de l'huile d'arbre à thé.

15. Utilisation d'un produit ou d'un sachet selon l'une quelconque des revendications 7 à 11, pour empêcher la prolifération de microorganismes à l'intérieur d'un bac de réception de déchets.
